(19) Europäisches Patentamt
European Patent Office
Office européen des brevets

(11) Veröffentlichungsnummer: **0 027 161**
**B1**

(12) # EUROPÄISCHE PATENTSCHRIFT

(45) Veröffentlichungstag der Patentschrift: **27.04.83**

(51) Int. Cl.³: **C 07 C 103/52,** A 61 K 37/26

(21) Anmeldenummer: **80104267.2**

(22) Anmeldetag: **19.07.80**

(54) **Insulinderivate, Verfahren zu ihrer Herstellung und ihre Verwendung.**

(30) Priorität: **27.07.79 DE 2930542**

(43) Veröffentlichungstag der Anmeldung: **22.04.81 Patentblatt 81/16**

(45) Bekanntmachung des Hinweises auf die Patenterteilung: **27.04.83 Patentblatt 83/17**

(84) Benannte Vertragsstaaten: **AT BE CH DE FR GB IT LI NL SE**

(56) Entgegenhaltungen:

**DE-A-2 038 121**
**DE-A-2 047 413**
**DE-A-2 433 883**
**US-A-3 960 830**

(73) Patentinhaber: **HOECHST AKTIENGESELLSCHAFT, Postfach 80 03 20, D-6230 Frankfurt/Main 80 (DE)**

(72) Erfinder: **Obermeier, Rainer, Dr., Langenhainer Weg 14, D-6234 Hattersheim am Main (DE)**
Erfinder: **Uhmann, Rainer, Dr., An der Landwehr 5, D-6239 Kriftel (DE)**
Erfinder: **Summ, Hans-Dieter, Dr., Heimchenweg 48, D-6230 Frankfurt am Main 80 (DE)**
Erfinder: **Regitz, Günther, Dr., Dachbergstrasse 68, D-6232 Bad Soden am Taunus (DE)**
Erfinder: **Geisen, Karl, Dr., Jahnstrasse 43, D-6000 Frankfurt am Main 71 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 027 161 B1**

ACTORUM AG

# Insulinderivate, Verfahren zu ihrer Herstellung und ihre Verwendung

Polyethylenglycol (PEG) wird häufig covalent mit Proteinen verbunden, um deren Hydrophilie zu steigern und die Antigenität zu verringern.

So ist in Arch. Biochem. Biophys die Reaktion von 2-Chlor-4-hydroxy-6-PEG mit einem Pollen-Allergen beschrieben.

In der niederländischen Patentanmeldung 7 409 770 wird über den Umsatz derselben Verbindung mit mehreren Enzymen und mit Insulin berichtet. Das resultierende Konjugat besitzt noch 50% Insulinaktivität, bezogen auf den Anteil des zugrunde liegenden Insulins.

Folgt man dem Beispiel 10 der niederländischen Patentanmeldung, so erhält man kein einheitliches Produkt. Ein Teil des Insulins ist an allen 3 Aminogruppen umgesetzt, ein Teil nur an zwei einer Aminogruppe. Ein ähnliches Ergebnis erhält man, wenn man einen aktivierten Bernsteinsäure-mono-PEGester, der ebenfalls in der genannten Anmeldung vorgeschlagen wird, mit Insulin umsetzt.

Über die Struktur der entstandenen Verbindungen erhält man durch einen Edman-Abbau Auskunft. Prüft man die Aminosäurezusammensetzung des Reaktionsprodukts (3 Phenylalaninreste) vor und nach Edman-Abbau, so stellt man fest, dass der Phenylalaningehalt von 3,0 auf etwa 2,5 zurückgegangen ist. Andererseits beträgt der durch saure Elektrophorese ermittelte Gehalt an dreifach substituiertem Insulin etwa 50%. Das bedeutet, dass die Aminogruppe des B1-Phenylalanins in mono- und disubstituierten Insulinen frei sein muss. Es sind also neben dem trisubstituierten Insulin Derivate entstanden, bei denen die Aminogruppen in A1 und B29 in diesen beiden Positionen gleichzeitig substituiert sind. Ein nur in B1-substituiertes Insulin liegt nicht vor. Dieser Befund deckt sich mit der aus Hoppe-Seyler's Z. physiol. Chem. *352* (1971), S. 1487 bekannten Reaktivität des Insulins, wonach in alkalischer Lösung bevorzugt die Aminogruppen in A1 und B29 reagieren, während die Aminogruppe in B1 nur in den trisubstituierten Derivaten in substituierter Form vorliegt.

Es wurde nun überraschend gefunden, dass man ein 100% wirksames PEG-substituiertes Insulin erhält, wenn man ein PEG-Derivat gezielt mit der Aminogruppe von B1-Phenylalanin umsetzt. Dieses Ergebnis ist deshalb überraschend, weil z.B. B1-Stearoylinsulin in vivo (Blutzuckersenkung am Kaninchen) wirkungslos ist.

Gegenstand der Erfindung sind somit Insulinderivate, welche dadurch gekennzeichnet sind, dass in ihnen die α-Aminogruppe der B-Kette des Insulins über ein Zwischenglied mit einem Monoalkylpolyethylenglycol verknüpft ist.

Besonders bevorzugt sind Derivate der allgemeinen Formel I

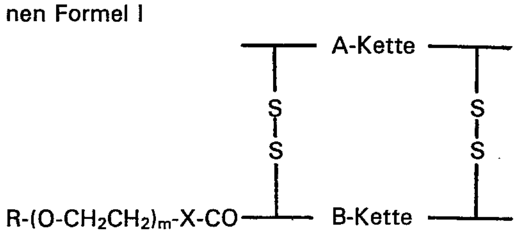

I

in der R Alkyl mit 1 bis 4 C-Atomen und X

-O-
$-O-CH_2-$
$-O-CO-(CH_2)_n-$ oder
$-O-CO-NH-(CH_2)_n-NH-$

bedeutet, wobei n 2 bis 8 und m 10 bis etwa 120 ist.

Gegenstand der Erfindung ist ferner ein Verfahren zur Herstellung von Insulinderivaten, das dadurch gekennzeichnet ist, dass man ein in den Positionen $N^{\alpha A1}$ und $N^{\varepsilon B29}$ mit Schutzgruppen versehenes Insulin mit einem Alkylpolyethylenglycol-Derivat umsetzt und danach die Schutzgruppen abspaltet.

So kann zur Herstellung von Insulinderivaten der allgemeinen Formel I $N^{\alpha A1}$, $N^{\varepsilon B29}$-Bis-tert.-butyloxycarbonyl-Insulin oder $N^{\alpha A1}$, $N^{\varepsilon B29}$-Bis-methylsulfonylethyloxycarbonyl-Insulin mit einem Alkylpolyethylenglycol-derivat der allgemeinen Formel II bzw. III umgesetzt

$R-(O-CH_2-CH_2)_m-X-CO-Y$ II

$R-(O-CH_2-CH_2)_m-O-CO-NH-(CH_2)_n-NCO$ III

in der R, X, m und n die obengenannte Bedeutung haben und Y Chlor, eine Aktivestergruppe oder Azid darstellt, und die tert.-Butyloxycarbonyl- bzw. Methylsulfonylethyloxycarbonyl-gruppen durch Behandeln mit Säure bzw. Lauge abgespalten werden.

An sich kann Monoalkyl-PEG über jeden beliebigen niedermolekularen Rest als Zwischenglied mit der α-Aminogruppe der B-Kette des Insulins verbunden sein, der die freie OH-Gruppe des Polyethylenglycols mit der Aminogruppe kovalent verknüpft. Der Charakter des Zwischengliedes beeinflusst die Eigenschaften der erfindungsgemässen Insulinderivate kaum.

So lassen sich neben den oben bereits genannten Zwischengliedern mit den allgemein bekannten Methoden der organischen Chemie, speziell der Peptidchemie, für -X- auch Zwischenglieder wie

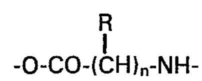

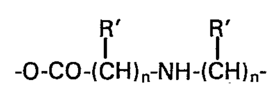

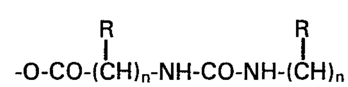 oder

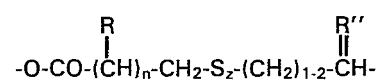

einführen. In diesen Formeln bedeutet z 1 oder 2.

Man kann auch anstelle der Esterbindung eine Amidbildung zum $R-(O-CH_2-CH_2)_m-$ herstellen, indem man gemäss der niederländischen Patentanmeldung 7 409 770 von dem entsprechenden $R-(O-CH_2-CH_2)_m-NH_2$ ausgeht. Über eine entsprechende

Halogenverbindung können mit Mercapto-Verbindungen auch Zwischenglieder der Struktur

$$-S-CH_2-(\underset{\displaystyle R'}{\underset{|}{C}}H)_p-$$

erhalten werden. In dieser Formel ist p eine ganze Zahl zwischen 1 und 8 und R' Wasserstoff oder Alkyl mit 1 bis 4 Kohlenstoffatomen. Wenn p = 1 ist, kann R' auch die Seitenkette einer natürlich vorkommenden α-Aminosäure, in der L-, D- oder DL-Form sein oder Acylamino bedeuten.

Auch heterocyclische Systeme, wie das bekannte 4-Hydroxy-1,3,5-triazin können als Zwischenglied dienen, wobei bei der genannten Verbindung die Vernetzungsstellen in 2- und 6-Position sind.

Ein Alkylpolyethylen-glycol-derivat der Formel III kann man erhalten durch Umsatz von $R-(O-CH_2-CH_2)_m-OH$ mit Hexamethylendiisocyanat. $R-(O-CH_2-CH_2)_m-O-CO-CH_2-CH_2-CO-ONSu$ (ONSu ist N-Hydroxy-succinimidyl) wird aus $R-(O-CH_2-CH_2)_m-OH$ mit Bernsteinsäureanhydrid und anschliessenden Umsatz mit Dicyclohexylcarbodiimid (DCC) und N-Hydroxysuccinimid (HONSu) hergestellt. $R-(O-CH_2-CH_2)_m-O-CH_2-CO-N_3$ erhält man aus $R-(O-CH_2-CH_2)_m-OH$ und Chloressigsäure-methylester, Umsetzung des Reaktionsprodukts mit Hydrazin und Überführen des Hydrazids in das Azid. $R-(O-CH_2-CH_2)_m-O-CO-Cl$ erhält man aus $R-(O-CH_2-CH_2)_m-OH$ mit überschüssigem Phosgen.

Mit tert.-Butyloxycarbonyl (Boc) oder Methylsulfonylethyloxycarbonyl (Msc) partiell geschützte Insulinderivate sind aus Hoppe-Seyler's Z. physiol. Chem. *352* (1971), S. 1487 und Chem. Ber. *108*(1975), S. 2758 bekannt.

Die Umsetzung der Alkylpolyethylen-glycol-derivate der allgemeinen Formeln II und III mit den partiell geschützten Insulinderivaten kann man in Lösungsmitteln wie Dimethylformamid oder Dimethylsulfoxid vornehmen. Auch Pyridin ist geeignet.

Zusatz einer tert.-Base wie Triäthylamin oder N-Methylmorpholin ist empfehlenswert.

Die Reaktion wird bevorzugt bei Raumtemperatur ablaufen gelassen, jedoch kann leichtes Erwärmen auf eine Temperatur unterhalb der Denaturierungstemperatur des Peptids förderlich sein.

Die Umsetzung wird so lange fortgesetzt, bis eine vollständige Acylierung eingetreten ist. Das lässt sich beispielsweise mit Hilfe einer papierelektrophoretischen Prüfung nach Hoppe-Seyler's Z. physiol. Chem. *352*(1971), S. 1487 feststellen.

Zur Abspaltung der Schutzgruppen dienen die in der Peptidchemie bekannten Verfahren.

So kann zur Abspaltung von Boc-Gruppen in einem geeigneten Mittel, zum Beispiel in Trifluoressigsäure gelöst und nach etwa 45 Minuten das Insulinderivat, z.B. mit Ether, ausgefällt werden.

Die Msc-Gruppe kann nach Chem. Ber. *108* (1975), S. 2758 durch kurzes Aufbewahren der Verbindung in alkalischem Dimethylformamid-Methanol-Wasser abgespalten werden. Die Verbindung wird danach durch Ansäuern, z.B. mit Essigsäure, und Ausfällen, z.B. mit Ether oder Essigsäure, isoliert.

Zur Entsalzung kann sich eine Filtration über eine Säule, z.B. aus Sephadex® -G15 oder Biogel P2 anschliessen. Sephadex ist ein vernetztes Dextran-Gel, Biogel ein vernetztes Polyacrylamid.

Eine chromatographische Reinigung der Verbindungen kann in der noch geschützten Form durch Verteilungschromatographie analog Hoppe-Seyler's Z. physiol. Chemie *354* (1973), S. 1285 vorgenommen werden.

Die Reinigung der Verbindungen kann auch nach Abspalten der Schutzgruppen durch Chromatographie, z.B. an Sephadex® G-50 oder Biogel P6 unter gleichzeitiger Entsalzung erfolgen.

Die neuen Insulinderivate dienen als Arzneimittel zur Behandlung des Diabetes Mellitus. Demgemäss ist Gegenstand der Erfindung auch ein Mittel, das eine der genannten Insulinderivate in wässriger Dispersion enthält und die Verwendung dieses Mittels zur Behandlung des Diabetes Mellitus.

Ein besonderer Vorzug der erfindungsgemässen Insulinderivate besteht darin, dass ihre Wirkung auf die Fettzelle stark reduziert ist. So zeigt z.B. B1-Methyl-PEG (1500)-succinoylinsulin bei der Blutzuckersenkung am Kaninchen über 100% Insulinwirkung, verglichen auf molarer Basis, während im Fettzelltest nur 65% gefunden werden. Die verstärkte und verlängerte Insulinwirkung der erfindungsgemässen Verbindungen gegenüber natürlichem Insulin wird besonders im Versuch an der adrenalektomierten Streptoratte deutlich.

Mit den neuen Insulinderivaten hat der Arzt erstmals Verbindungen in der Hand, die die bekannte anabole Insulinwirkung bevorzugt am Muskelgewebe und weniger am Fettgewebe entfalten. Damit ist ein schon lange gesuchter Wirkungssplit erreicht.

Darüber hinaus besitzen die erfindungsgemässen Insulinderivate eine stark verminderte Antigenität.

Die nachfolgenden Beispiele erläutern die Herstellung der neuen Verbindungen. Als Ausgangsmaterial können Insuline verschiedener Spezies wie Rind, Schwein oder Mensch dienen.

*Beispiel 1*

*B1-Methyl-PEG(1500)-carbonylamino-hexamethylenaminocarbonyl-insulin (Rind)*

a) *Monoisocyanat aus Methylpolyäthylenglycol (1500) und Hexamethylendiisocyanat*

15 g Monomethylpolyäthylenglykol (MG 1500) werden in 200 ml Benzol in der Wärme gelöst. Die Lösung wird mit 1 ml Dibutylzinnlaurat und 8,6 ml Hexamethylendiisocyanat versetzt und 4 Stunden unter Rückfluss erhitzt. Nach dem Erkalten wird das Produkt durch Zusatz von Petroläther gefällt, der Überstand dekantiert, der Feststoff in Toluol gelöst und die Lösung erneut mit Petroläther versetzt. Die Fällung wird noch zweimal wiederholt, das Produkt wird abgesaugt und im Vakuum getrocknet. Ausbeute: 14,6 g; Charakterisierung durch IR-Spektrum (1705 $cm^{-1}$ Amid; 2260 $cm^{-1}$ Isocyanat).

b) 200 mg $N^{\alpha A1}$, $N^{\varepsilon B29}$-Bis-Boc-insulin vom Rind, hergestellt nach der DPS 2 162 164, werden in 3 ml Dimethylformamid, das 0.2 ml N-Ethylmorpholin enthält, mit 400 mg der oben hergestellten Verbindung 18 Stunden bei Raumtemperatur gerührt. Man fällt mit Ether einen Niederschlag

aus und wäscht ihn mit Ether und Essigester. Ausbeute nach dem Trocknen im Vak. 278 mg. Da das Produkt elektrophoretisch hinreichend einheitlich ist, kann auf eine chromatographische Reinigung verzichtet werden.

Zur Abspaltung der Schutzgruppen löst man das Produkt in 2 ml Trifluoressigsäure und fällt nach 45 Minuten Stehen bei Raumtemperatur mit Ether 243 mg Reaktionsprodukt aus. Nach Lösen des Präzipitats in 2 ml 0,1 M Ammonacetat/0,1 N Essigsäure wird die Lösung über eine Säule Sephadex® -G-50 1 × 50 cm mit 0,1 M Ammonacetat/0,1 N Essigsäure chromatographiert. Ausbeute: 218 mg.

Die Verbindung ist in der Papierelektrophorese bei pH 2 einheitlich und verhält sich erwartungsgemäss wie ein monoacyliertes Insulin.

*Beispiel 2*

*B1-Methyl-PEG- (500)-succinoyl-insulin (Schwein)*

a) *Bernsteinsäure-mono-methylpolyäthylenglycolester (500)*

15 g Mono-methylpolyäthylenglycol (500) werden in 40 ml Methylenchlorid gelöst, 5 g Bernsteinsäureanhydrid zugesetzt und über Nacht bei Raumtemperatur gerührt. Danach wird das Reaktionsgemisch noch 1 Stunde am Rückfluss gekocht. Nach Erkalten wird das Lösungsmittel im Vakuum entfernt und der Rückstand in 5 g-Portionen in Methanol gelöst und über Sephadex LH 20 in Methanol chromatographiert. Die Substanz wird im Dünnschichtchromatographie-System Methylenchlorid/Diäthylenglycolmonomethyläther/Pyridin (85:15:2 v/v) auf Reinheit überprüft. IR-Spektrum: 1740 $cm^{-1}$.

b) *Bernsteinsäure-mono-methylpolyäthylenglycolester (500)-mono-N-hydroxysuccinimidester*

1,8 g (3 mmol) Bernsteinsäure-mono-methylpolyäthylenglycolester (500) werden in 20 ml Tetrahydrofuran gelöst und der Lösung 345 mg (3 mmol) N-Hydroxysuccinimid und 620 mg DCC zugefügt. Die Mischung wird 24 Stunden bei Raumtemperatur gerührt und danach vom ausgefallenen Niederschlag befreit. Sodann wird das Lösungsmittel im Vakuum entfernt, das zurückbleibende Öl mit Äther überschichtet und zur Kristallisation auf $-20°C$ abgekühlt. Der Überstand wird abdekantiert und der kristalline Rückstand auf Raumtemperatur erwärmt, wobei die Kristalle wieder zerfliessen. Diese Kristallisation wird wiederholt. Die Substanz kann unter trockenem Äther aufbewahrt werden. Ausbeute: 1,6 g. - Charakterisierung durch IR-Spektrum (1820, 1890, 1740 $cm^{-1}$).

c) *B1-Methyl-PEG (500)-succinoyl-insulin (Schwein)*

3,0 g $N^{\alpha A1}$, $N^{\varepsilon B29}$-Bis-Msc-insulin vom Schwein, hergestellt nach Chem. Ber. *108* (1975), S. 2758, werden in 40 ml Dimethylformamid und 0,09 ml N-Ethylmorpholin gelöst. Man gibt 1,0 g Methyl-PEG (500)-Succinoyl-ONSu zu und rührt über Nacht bei Raumtemperatur.

Zur Aufarbeitung versetzt man mit 0,1 ml Essigsäure, fällt das Reaktionsprodukt mit Ether und wäscht mit Methylenchlorid und Ether. Ausbeute: 4,0 g.

Die Reinigung erfolgt durch Verteilungschromatographie analog Hoppe Seyler's Z. physiol. chem. *354* (1973), S. 1285 in einer Säule 200 × 6 cm. Die Insulinkonzentration im Eluat wird über Messung der UV-Extinktion bei 254 nm verfolgt. Die Fraktionen des Hauptpeaks, die chromatographisch einheitlich sind [Dünnschichtchromatographie aus Kieselgel. Laufmittel Chloroform/Methanol/Wasser/Eisessig (60: 45:14:3)] werden zusammengefasst. Ausbeute: 2,1 g.

Zur Abspaltung der Msc-Schutzgruppen löst man die vom Lösungsmittel befreite Substanz in 10 ml Dimethylformamid und versetzt bei 0°C unter Rühren mit 10 ml Methanol und 20 ml 1 N NaOH. Nach 45 sek. gibt man 20 ml 1 N Essigsäure zu und fällt mit Essigester/Ether (1:1) 2,0 g Insulinderivat aus, das analog Beispiel 2 durch Gelfiltration entsalzt wird. Ausbeute: 1,85 g.

*Beispiel 3*

*B1-Methyl-PEG(1500)-succinoyl-insulin (Schwein)*

3,5 g $N^{\alpha A1}$, $N^{\varepsilon B29}$-Bis-Boc-insulin (Schwein) werden in einer Lösung von 40 ml Dimethylformamid und 0,09 ml N-Ethylmorpholin mit 8,0 g Methyl-PEG(1500)-succinoyl-ONSu, hergestellt analog Beispiel 2, über Nacht bei Raumtemperatur gerührt.

Zur Aufarbeitung versetzt man mit 0,1 ml Essigsäure, fällt das Reaktionsprodukt mit Ether und wäscht es mit Methylenchlorid und Ether. Ausbeute: 4,5 g. Die Reinigung durch Verteilungschromatographie wird analog Beispiel 2 ausgeführt. Ausbeute: 2,8 g.

Man spaltet wie bei Beispiel 1 die Schutzgruppen ab und entsalzt wie bei Beispiel 2 angegeben. Ausbeute: 2,5 g. Die Reinheit der Verbindung in der Polyacrylamidgel-Elektrophorese entspricht der von chromatographisch gereinigtem Insulin.

**Patentansprüche für die Vertragsstaaten:**
BE, CH, DE, FR, GB, IT, LI, NL, SE

1. Insulinderivat, dadurch gekennzeichnet, dass die Aminogruppe des B1-Phenylalanins über einen niedermolekularen Rest als Zwischenglied mit der freien OH-Gruppe eines Monoalkylpolyethylenglycols kovalent verknüpt ist.

2. Insulinderivat der Formel I

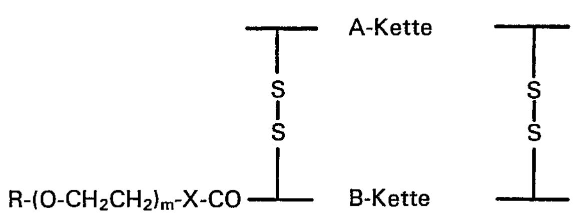

in der R Alkyl mit 1 bis 4 Kohlenstoffatomen und X

-O-
$-O-CH_2-$
$-O-CO-(CH_2)_n-$ oder
$-O-CO-NH-(CH_2)_n-NH-$

bedeutet, wobei n 2 bis 8 und m 10 bis etwa 120 ist.

3. Verfahren zur Herstellung eines Insulinderivats, dadurch gekennzeichnet, dass man ein in den Positionen $N^{\alpha A1}$ und $N^{\varepsilon B29}$ mit Schutzgruppen versehenes Insulin mit einem Alkylpolyethylenglycol-Derivat umsetzt und danach die Schutzgruppen abspaltet.

4. Verfahren zur Herstellung eines Insulinderivats der Formel I, dadurch gekennzeichnet, dass man $N^{\alpha A1}$, $N^{\varepsilon B29}$-Bis-tert.-butyloxycarbonyl-Insulin oder $N^{\alpha A1}$, $N^{\varepsilon B29}$-Bis-methylsulfonylethyloxycarbonyl-insulin mit einem Alkylpolyethylenglycol-Derivat der allgemeinen Formel II bzw. III umsetzt

$R\text{-}(O\text{-}CH_2\text{-}CH_2)_m\text{-}X\text{-}CO\text{-}Y$ II

$R\text{-}(O\text{-}CH_2\text{-}CH_2)_m\text{-}O\text{-}CO\text{-}NH\text{-}(CH_2)_n\text{-}NCO$ III

in der R, X, m und n die obengenannte Bedeutung haben und Y Chlor, eine Aktivestergruppe oder Azid darstellt und die tert.-Butyloxycarbonyl- bzw. Methylsulfonylethyloxycarbonyl-gruppen durch Behandeln mit Säure bzw. Lauge abspaltet.

5. Mittel, enthaltend ein Insulinderivat gemäss Anspruch 1 in wässriger Dispersion.

6. Verbindung gemäss Anspruch 1 zur Verwendung als Heilmittel zur Behandlung des Diabetes mellitus.

**Patentansprüche für den Vertragstaat:** AT

1. Verfahren zur Herstellung eines Insulinderivats dessen α-Aminogruppe der B-Kette des Insulins über ein Zwischenglied mit Monoalkylpolyethylenglycol verknüpft ist, dadurch gekennzeichnet, dass man ein in den Positionen $N^{\alpha A1}$ und $N^{\varepsilon B29}$ mit Schutzgruppen versehenes Insulin mit einem Alkylpolyethylenglycol-Derivat umsetzt und danach die Schutzgruppen abspaltet.

2. Verfahren nach Anspruch 1, dadurch gekennzeichnet, dass man ein Insulinderivat der Formel I

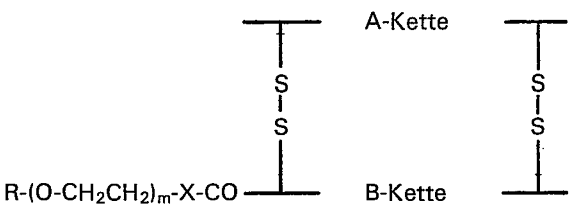

in der R Alkyl mit 1 bis 4 Kohlenstoffatomen und X

-O-
$-O\text{-}CH_2-$
$-O\text{-}CO\text{-}(CH_2)_n-$ oder
$-O\text{-}CO\text{-}NH\text{-}(CH_2)_n\text{-}NH-$

bedeutet, wobei n 2 bis 8 und m 10 bis etwa 120 ist, herstellt.

3. Verfahren zur Herstellung eines Insulinderivats der Formel I, dadurch gekennzeichnet, dass man $N^{\alpha A1}$, $N^{\varepsilon B29}$-Bis-tert.-butyloxycarbonyl-Insulin oder $N^{\alpha A1}$, $N^{\varepsilon B29}$-Bis-methylsulfonylethyloxycarbonyl-insulin mit einem Alkylpolyethylenglycol-Derivat der allgemeinen Formel II bzw. III umsetzt

$R\text{-}(O\text{-}CH_2\text{-}CH_2)_m\text{-}X\text{-}CO\text{-}Y$ II

$R\text{-}(O\text{-}CH_2\text{-}CH_2)_m\text{-}O\text{-}CO\text{-}NH\text{-}(CH_2)_n\text{-}NCO$ III

in der R, X, m und n die obengenannte Bedeutung haben und Y Chlor, eine Aktivestergruppe oder Azid darstellt und die tert.-Butyloxycarbonyl- bzw. Methylsulfonylethyloxycarbonyl-gruppen durch Behandeln mit Säure bzw. Lauge abspaltet.

**Revendications pour les Etats contractants:** BE, CH, LI, DE, FR, GB, IT, NL, SE

1. Dérivé d'insuline, caractérisé en ce que le groupe amino de la phénylalanine en B1 est relié de manière covalente au groupe OH libre d'un monoalcoylpolyéthylène glycol au moyen d'un radical à faible masse moléculaire servant d'élément intermédiaire.

2. Dérivé d'insuline de formule I

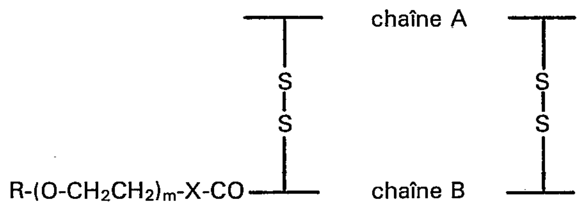

dans laquelle R représente un groupe alcoyle ayant de 1 à 4 atomes de carbone et X est

-O-
$-O\text{-}CH_2-$
$-O\text{-}CO\text{-}(CH_2)_n-$ ou
$-O\text{-}CO\text{-}NH\text{-}(CH_2)_n\text{-}NH-$

n étant compris entre 2 et 8 et m étant compris entre 10 et 120 environ.

3. Procédé pour la préparation d'un dérivé d'insuline, caractérisé en ce qu'on fait réagir une insuline ayant des groupes protecteurs en les positions $n^{\alpha A1}$ et $N^{\varepsilon B29}$ avec un dérivé d'alcoylpolyéthylène glycol, puis on élimine les groupes protecteurs.

4. Procédé pour la préparation d'un dérivé d'insuline de formule I, caractérisé en ce qu'on fait réagir la $N^{\alpha A1}$, $N^{\varepsilon B29}$-bis-tert.-butyloxycarbonyl-insuline ou la $N^{\alpha A1}$, $N^{\varepsilon B29}$-bis-méthylsulfonyléthylcarbonyl-insuline avec un dérivé d'alcoylpolyéthylène glycol de formule générale II ou III

$R\text{-}(O\text{-}CH_2\text{-}CH_2)_m\text{-}X\text{-}CO\text{-}Y$ II

$R\text{-}(O\text{-}CH_2\text{-}CH_2)_m\text{-}O\text{-}CO\text{-}NH\text{-}(CH_2)_n\text{-}NCO$ III

dans lesquelles R, X, m et n ont les significations ci-dessus et Y représente le chlore, un groupe ester actif ou un azide et on élimine les groupes tert.-butyloxycarbonyle ou méthylsulfonyléthylcarbonyle par le traitement avec un acide ou une base.

5. Agent contenant un derivé d'insuline selon la revendication 1, en dispersion aqueuse.

6. Composé selon la revendication 1, destiné à être utilisé comme médicament pour le traitement du diabète sucré.

**Revendications pour l'Etat contranctant:** AT

1. Procédé pour la préparation d'un dérivé d'insuline dont le groupe α-amino de la chaîne B de l'insuline est relié par un élément intermédiaire à un monoalcoylpolyéthylène glycol, caractérisé en ce qu'on fait réagir une insuline possédant des groupes protecteurs dans les positions $N^{\alpha A1}$ et $N^{\varepsilon B29}$ avec un dérivé d'alcoylpolyéthylène glycol et on élimine ensuite les groupes protecteurs.

2. Procédé selon la revendication 1, caractérisé en ce qu'on prépare un dérivé d'insuline de formule I

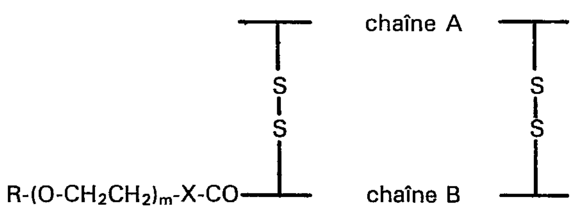

dans laquelle R présente un group alcoyle ayant de 1 à 4 atomes de carbone et X est

$-O-$
$-O-CH_2-$
$-O-CO-(CH_2)_n-$ ou
$-O-CO-NH-(CH_2)_n-NH-$

n étant compris entre 2 et 8 et m étant compris entre 10 et 120 environ.

3. Procédé pour la préparation d'un dérivé d'insuline de formule I, caractérisé en ce qu'on fait réagir une $N^{\alpha A1}$, $N^{\varepsilon B29}$-bis-tert.-butyloxycarbonyl-insuline ou une $N^{\alpha A1}$, $N^{\varepsilon B29}$-bis-méthylsulfonyléthylcarbonyl-insuline avec un dérivé d'alcoylpolyéthylène glycol de formule générale II ou III

$R-(O-CH_2-CH_2)_m-X-CO-Y$ II

$R-(O-CH_2-CH_2)_m-O-CO-NH-(CH_2)_n-NCO$ III

dans lesquelles R, X, m et n ont les significations ci-dessus et Y représente le chlore, un groupe ester actif ou un azide et on élimine les groupes tert.-butyloxycarbonyle ou méthylsulfonyléthylcarbonyle par le traitement avec un acide ou une base.

**Claims for the Contracting States:**
BE, CH, LI, DE, FR, GB, IT, NL, SE

1. Insulin derivative wherein the amino group of the B1-phenylalanine is linked to the free OH group of a monoalkylpolyethyleneglycol via a low molecular radical connecting member.

2. Insulin derivative of the formula I

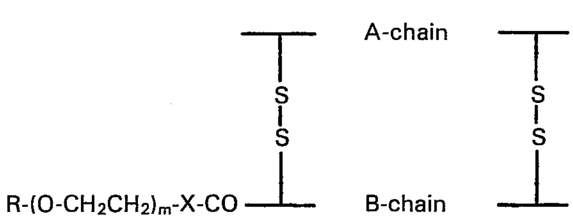

in which R is alkyl having from 1 to 4 carbon atoms, X is

$-O-$
$-O-CH_2-$
$-O-CO-(CH_2)_n-$ or
$-O-CO-NH-(CH_2)_n-NH-$

n is an integer of from 2 to 8, and m an integer of from 10 to about 120.

3. A process for the preparation of an insulin derivative, which comprises reacting an insulin containing protecting groups in the $N^{\alpha A1}$ and $n^{\varepsilon B29}$ positions with an alkylpolyethyleneglycol derivative, and subsequently splitting off the protecting groups.

4. A process for the preparation of an insulin derivative of the formula I as claimed in claim 2, which comprises reacting $N^{\alpha A1}$, $N^{\varepsilon B29}$-bis-tert.-butyloxycarbonyl-insulin or $N^{\alpha A1}$, $N^{\varepsilon B29}$-bis-methylsulfonylethyloxycarbonyl-insulin with an alkylpolyethyleneglycol derivative of the formulae II or III

$R-(O-CH_2-CH_2)_m-X-CO-Y$ II

$R-(O-CH_2-CH_2)_m-O-CO-NH-(CH_2)_n-NCO$ III

in which R, X, m and n are as defined above and Y is chlorine, an active ester group or an azide, and splitting off the tert.-butyloxycarbonyl or methylsulfonylethyloxycarbonyl groups by treatment with acid or alkali.

5. Medicament containing an insulin derivative as claimed in claim 1 in aqueous dispersion.

6. Compound according to claim 1 for use as a medicament for treating diabetes mellitus.

**Claims for Contracting State:** AT

1. A process for the preparation of an insulin derivative wherein the alpha-amino group of the insulin B-chain is linked to a monoalkylpolyethyleneglycol via a connecting member, which process comprises reacting an insulin containing protecting groups in the $N^{\alpha A1}$ and $N^{\varepsilon B29}$ positions with an alkyl-polyethyleneglycol derivative and subsequently splitting off the protecting groups.

2. A process according to claim 1, which comprises reacting an insulin derivative of the formula I

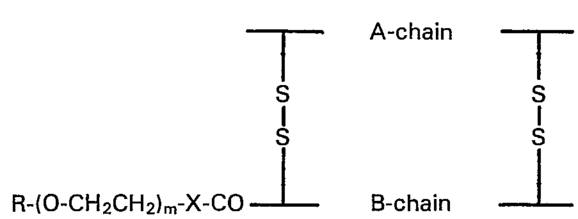

in which R is alkyl having from 1 to 4 carbon atoms, X is

$-O-$
$-O-CH_2-$
$-O-CO-(CH_2)_n-$ or
$-O-CO-NH-(CH_2)_n-NH-$

n is an integer of from 2 to 8, and m an integer of from 10 to about 120.

3. A process for the preparation of an insulin derivative of the formula I, which comprises reacting $N^{\alpha A1}$, $N^{\varepsilon B29}$-bis-tert.-butyloxycarbonyl-insulin or $N^{\alpha A1}$, $N^{\varepsilon B29}$-bis-methylsulfonylethyloxycarbonyl-insulin with an alkylpolyethyleneglycol derivative of the formulae II or III

$$R\text{-}(O\text{-}CH_2\text{-}CH_2)_m\text{-}X\text{-}CO\text{-}Y \quad II$$

$$R\text{-}(O\text{-}CH_2\text{-}CH_2)_m\text{-}O\text{-}CO\text{-}NH\text{-}(CH_2)_n\text{-}NCO \quad III$$

in which R, X, m and n are as defined above and Y is chlorine, an active ester group or an azide, and splitting off the tert.-butyloxycarbonyl or methylsulfonylethyloxycarbonyl groups by treatment with acid or alkali.